# EUROPEAN PATENT APPLICATION

(11) **EP 2 025 662 A1**
(43) Date of publication of application: **18.02.2009**
(21) Application number: 07742698.9
(22) Date of filing: 27.04.2007
(51) Int. Cl.: C07C 43/23

(54) **METHOD FOR PURIFICATION OF REDUCED COENZYME Q10**

(30) Priority: 28.04.2006 JP 2006126900
(71) Applicant: Kaneka Corporation, Kita-ku Osaka-shi, Osaka 530-8288 (JP)
(72) Inventor: UEDA, Takahiro, Kobe-shi, Hyogo 655-0872 (JP); ONO, Tadao, Kobe-shi, Hyogo 655-0872 (JP); KITAMURA, Shiro, Akashi-shi, Hyogo 673-0882 (JP)
(74) Representative: HOFFMANN EITLE
(86) International application number: PCT/JP2007/059263
(87) International publication number: WO 2007/126086

(57) **Abstract**

The present invention provides reduced coenzyme 0₁₀ with a low reduced coenzyme Q₁₀ analog content, which is useful as food, food with nutrient function claims, food for specified health use, nutritional product, nutritional supplement, animal drug, drink, feed, cosmetic, pharmaceutical product, therapeutic drug, prophylactic drug and the like, and a production method of the reduced coenzyme Q₁₀. The present invention also provides a production method of reduced coenzyme Q₁₀ which includes subjecting reduced coenzyme Q₁₀ to chromatography under oxidation preventive conditions, or purifying oxidized coenzyme Q₁₀ by chromatography and converting the oxidized coenzyme Q₁₀ to reduced coenzyme Q₁₀. According to this method, high quality reduced coenzyme Q₁₀ containing not more than 1 wt% of at least one kind selected from the group consisting of cis-reduced coenzyme Q₁₀, reduced coenzyme Q₁₁ and ubichromenol can be provided.

## Description

### Technical Field of the Invention

The present invention relates to a reduced coenzyme Q₁₀ crystal or composition having a low content of reduced coenzyme Q₁₀ analogs such as a cis form of reduced coenzyme Q₁₀ (hereinafter cis-reduced coenzyme Q₁₀), reduced coenzyme Q₁₁. ubichromenol and the like, and production methods thereof. As compared to oxidized coenzyme Q₁₀, reduced coenzyme Q₁₀ shows high oral absorbability, and is a compound useful as a superior food, food with nutrient function claims, food for specified health use, nutritional supplement, nutritional product, animal drug, drink, feed, pet food, cosmetic, pharmaceutical product, therapeutic drug, prophylactic drug and the like.

### Background Art

Coenzyme Q₁₀ is locally present in mitochondria, lysosome, Golgi body, microsome, peroxisome, cell membrane and the like and is a substance indispensable to the functional maintenance of the body. It is known to be involved in the activation of ATP production as a constituent component of the electron transport system in mitochondria, antioxidant action in the body and membrane stabilization, and is used for food, pharmaceutical agent, cosmetic and the like. The coenzyme Q₁₀ includes oxidized type and reduced type. It is known that reduced coenzyme Q₁₀ is obtained by purifying, according to a known method, a reduced coenzyme Q₁₀ obtained by a method such as synthesis, fermentation, extraction from a naturally occurring substance and the like (patent reference 1). Patent reference 1 describes that, in this case, oxidized coenzyme Q₁₀ contained in the above-mentioned reduced coenzyme Q₁₀ can be reduced with a general reducing agent such as sodium borohydride, sodium dithionite (sodium hydrosulfite) and the like, and purified, and that the reduced coenzyme Q₁₀ can also be obtained by a method comprising reacting existing highly pure coenzyme Q₁₀ with the above-mentioned reducing agent.

When reduced coenzyme Q₁₀ is obtained by the aforementioned synthesis, fermentation, extraction from a naturally occurring substance and the like, various reduced coenzyme Q₁₀ analogs (e.g., cis-reduced coenzyme Q₁₀, ubichromenol, reduced coenzyme Q₁₁ etc.) are contained therein. In general, a method including dissolving a mixture of the object product and impurity in a solvent and then crystallizing the object product from the solution (crystal precipitation method) is effective for the removal of impurity. For example, a method of removing reduced coenzyme Q₉, reduced coenzyme Q₈ and reduced coenzyme Q₇, which are reduced coenzyme Q₁₀ analogs, from reduced coenzyme Q₁₀ by the crystal precipitation method is already known (patent references 2, 3).
(patent reference 1) JP-A-10-109933
(patent reference 2) W003/6408
(patent reference 3) W003/6409

### Disclosure of the Invention

### Problems to be Solved by the Invention

According to the investigation of the present inventors, it was clarified that cis-reduced coenzyme Q₁₀, reduced coenzyme Q₁₁, ubichromenol and the like, from among reduced coenzyme Q₁₀ analogs, could not be easily removed by conventional methods such as crystal precipitation method and the like.
In view of the above-mentioned situation, the present invention aims at provision of a production method of reduced coenzyme Q₁₀, which is capable of conveniently removing the above-mentioned analogs, and high quality reduced coenzyme Q₁₀ having a low content of the analogs.

### Means of Solving the Problems

The present inventors have conducted intensive studies in an attempt to solve the above-mentioned problems and found that analogs of reduced coenzyme Q₁₀, which are generally difficult to remove, can be efficiently removed under particular chromatography conditions, and a substantially pure reduced coenzyme Q₁₀ crystal having a low content of a cis-reduced coenzyme Q₁₀, reduced coenzyme Q₁₁, and ubichromenol and the like can be obtained, which resulted in the completion of the present invention.

Accordingly, the present invention provides the following.
[1] A substantially pure reduced coenzyme Q₁₀ crystal wherein the content of at least one kind of reduced coenzyme Q₁₀ analog selected from the group consisting of cis-reduced coenzyme Q₁₀, reduced coenzyme Q₁₁, and ubichromenol is not more than 1 wt%.
[2] The reduced coenzyme Q₁₀ crystal of [1], wherein the content of cis-reduced coenzyme Q₁₀ is not more than 1 wt%.
[3] The reduced coenzyme Q₁₀ crystal of [1], wherein the content of reduced coenzyme Q₁₁ is not more than 1 wt%.
[4] The reduced coenzyme Q₁₀ crystal of [1], wherein the content of ubichromenol is not more than 1 wt%.
[5] The reduced coenzyme Q₁₀ crystal of any one of [1] to [4], wherein the total content of cis-reduced coenzyme Q₁₀, reduced coenzyme Q₁₁, and ubichromenol is not more than 3 wt%.
[6] The reduced coenzyme Q₁₀ crystal of [1], wherein the content of each of cis-reduced coenzyme Q₁₀, reduced coenzyme Q₁₁, and ubichromenol is not more than 1 wt%.
[7] A reduced coenzyme Q₁₀-containing composition wherein the content of at least one kind of reduced coenzyme Q₁₀ analog selected from the group consisting of cis-reduced coenzyme Q₁₀, reduced coenzyme Q₁₁, and ubichromenol is not more than 1 wt%.
[8] The reduced coenzyme Q₁₀-containing composition of [7], wherein the content of cis-reduced coenzyme Q₁₀ is not more than 1 wt%.
[9] The reduced coenzyme Q₁₀-containing composition of [7], wherein the content of reduced coenzyme Q₁₁ is not more than 1 wt%.
[10] The reduced coenzyme Q₁₀-containing composition of [7], wherein the content of ubichromenol is not more than 1 wt%.
[11] The reduced coenzyme Q₁₀-containing composition of any one of [7] to [10], wherein the total content of cis-reduced coenzyme Q₁₀, reduced coenzyme Q₁₁, and ubichromenol is not more than 3 wt%.
[12] The reduced coenzyme Q₁₀ crystal of [7], wherein the content of each of cis-reduced coenzyme Q₁₀, reduced coenzyme Q₁₁, and ubichromenol is not more than 1 wt%.
[13] A production method of the reduced coenzyme Q₁₀ crystal of [1] or the reduced coenzyme Q₁₀-containing composition of [7], which comprises a step for removing impurities by chromatography.
[14] The production method of [13], wherein the chromatography is column chromatography.
[15] The production method of [13] or [14], wherein a carrier of the chromatography is silica gel or chemically-modified silica gel.
[16] The production method of [15], wherein the chemically-modified silica gel is silica gel modified by an octadecyl group.
[17] The production method of any one of [13] to [16], which comprises a step of purifying reduced coenzyme Q₁₀ containing at least one kind of cis-reduced coenzyme Q₁₀, reduced coenzyme Q₁₁, or ubichromenol as impurity by chromatography.
[18] The production method of [17], wherein the chromatography is performed under an environment where reduced coenzyme Q₁₀ is protected from oxidation.
[19] The production method of [17] or [18], wherein cis-reduced coenzyme Q₁₀ is removed using a mixed solvent of hexane, 2-propanol and acetic acid as a developing solvent.
[20] The production method of [17] or [18], wherein the developing solvent is any one of hexane, methanol, ethanol and a mixed solvent thereof, and reduced coenzyme Q₁₁ and/or ubichromenol are/is removed.
[21] The production method of any one of [13] to [16], which comprises purifying oxidized coenzyme Q₁₀ containing at least one kind of cis-oxidized coenzyme Q₁₀, oxidized coenzyme Q₁₁, or ubichromenol as impurity by chromatography, and converting the purified oxidized coenzyme Q₁₀ to reduced coenzyme Q₁₀.
[22] The production method of [21], wherein the cis-oxidized coenzyme Q₁₀ is removed using, as the developing solvent, a mixed solvent of two or more kinds selected from hexane, 2-propanol and ethyl acetate.
[23] The production method of [21], wherein the oxidized coenzyme Q₁₁ and/or ubichromenol are/is removed using, as the developing solvent, any one of hexane, methanol, ethanol and a mixed solvent thereof.

### Effect of the Invention

According to the present invention, analogs of reduced coenzyme Q₁₀, which are generally difficult to remove, can be removed conveniently and high quality reduced coenzyme Q₁₀ can be economically obtained with good workability.
Particularly, when reduced coenzyme Q₁₀ is added to food, pharmaceutical product and the like, a decrease in the absolute amount of reduced coenzyme Q₁₀, which is the active ingredient, can be suppressed by decreasing the content of reduced coenzyme Q₁₀ analogs such as cis-reduced coenzyme Q₁₀, reduced coenzyme Q₁₁, ubichromenol and the like, whereby an unpredictable effect caused by ingestion of impurities generally absent in the human body can be eliminated. While a long-term intake of a large amount of reduced coenzyme Q₁₀ as a food and the like has not been known conventionally, in such a case, eradication of the aforementioned adverse effect of impurities is particularly effective.

### Best Mode for Practicing the Invention

The present invention is explained in detail in the following. In the present specification, when simply expressed as coenzyme Q₁₀, it includes an oxidized form, a reduced form and a mixture thereof when they are both present. When trans or cis is not indicated, all-trans type coenzyme Q₁₀ is intended.

The reduced coenzyme Q₁₀ to be used in the present invention can be obtained, for example, by a conventionally known method such as synthesis, fermentation, extraction from a naturally occurring substance and the like. Preferably, it is oxidized coenzyme Q₁₀ contained in reduced coenzyme Q₁₀ obtained by synthesis, fermentation, extraction from a naturally occurring substance and the like, or obtained by reduction of oxidized coenzyme Q₁₀, more preferably, obtained by reduction of oxidized coenzyme Q₁₀ with a reducing agent such as ascorbic acid, an ester thereof or a salt thereof, sodium hyposulfite (sodium dithionite) and the like.

The reduced coenzyme Q₁₀ crystal of the present invention (sometimes to be referred to as the crystal of the present invention) is a substantially pure reduced coenzyme Q₁₀ crystal wherein the content of at least one kind of reduced coenzyme Q₁₀ analogs selected from the group consisting of cis-reduced coenzyme Q₁₀, reduced coenzyme Q₁₁ and ubichromenol is not more than 1 wt%.

In the crystal of the present invention, the content of cis-reduced coenzyme Q₁₀ is generally not more than 1 wt%, preferably not more than 0.8 wt%, more preferably not more than 0.5 wt%. By removing cis-reduced coenzyme Q₁₀, a decrease in the absolute amount of reduced coenzyme Q₁₀, which is the active ingredient, can be suppressed and an unpredictable influence caused by ingestion of impurities generally absent in the human body can be eliminated. In particular, since cis-reduced coenzyme Q₁₀ may not fully exhibit its antioxidant activity due to the steric structure thereof and may act as a prooxidant, the risk of developing an oxidation stress can also be obliterated by removing cis-reduced coenzyme Q₁₀.

In the crystal of the present invention, the content of reduced coenzyme Q₁₁ is generally not more than 1 wt%, preferably not more than 0.8 wt%, more preferably not more than 0.5 wt%. By removing reduced coenzyme Q₁₁, a decrease in the absolute amount of reduced coenzyme Q₁₀, which is the active ingredient, can be suppressed and an unpredictable influence caused by ingestion of an ingredient not generally biosynthesized in the human body can be eliminated.

In the crystal of the present invention, the content of ubichromenol is generally not more than 1 wt%, preferably not more than 0.8 wt%, more preferably not more than 0.5 wt%. By removing ubichromenol, a decrease in the absolute amount of reduced coenzyme Q₁₀, which is the active ingredient, can be suppressed and an unpredictable influence caused by ingestion of impurities generally absent in the human body can be eliminated.

In the crystal of the present invention, the total content of cis-reduced coenzyme Q₁₀, reduced coenzyme Q₁₁ and ubichromenol is generally not more than 3 wt%, preferably not more than 2 wt%, more preferably not more than 1 wt%. When the content of the above-mentioned analog is within the above-mentioned preferable range, for example, a decrease in the absolute amount of reduced coenzyme Q₁₀, which is the active ingredient, can be suppressed and an unpredictable influence caused by ingestion of impurities generally absent in the human body can be eliminated. Therefore, when the content of the above-mentioned analog is within the above-mentioned preferable range, for example, a long-term, safe use can be ensured even when the crystal of the present invention is consumed in a large amount every day in food, supplement and the like.

In addition, using high quality reduced coenzyme Q₁₀, or a crystal thereof, obtained by the below-mentioned production method as an active ingredient, a high quality reduced coenzyme Q₁₀-containing composition with a low content of reduced coenzyme Q₁₀ analog can be obtained.
That is, the reduced coenzyme Q₁₀-containing composition of the present invention (to be also referred to as the composition of the present invention) includes reduced coenzyme Q₁₀ (crystal) wherein the content of at least one kind of reduced coenzyme Q₁₀ analog selected from the group consisting of cis-reduced coenzyme Q₁₀, reduced coenzyme Q₁₁, and ubichromenol is not more than 1 wt%.

The preferable range of cis-reduced coenzyme Q₁₀, reduced coenzyme Q₁₁, and ubichromenol is the same as that mentioned for the above-mentioned crystal of the present invention. When the composition of the present invention is similarly consumed in a large amount every day, for example, in a supplement, since a decrease in the absolute amount of reduced coenzyme Q₁₀, which is the active ingredient, can be suppressed and an unpredictable influence caused by ingestion of impurities generally absent in the human body can be eliminated, a long-term, safe administration can be ensured.

While the composition of the present invention can be used as it is, it may be processed into a dosage form for oral administration such as capsule (microcapsule, hard capsule, soft capsule), tablet, syrup, drink and the like and used preferably. It can be processed into a dosage form for parenteral administration such as cream, suppository, toothpaste and the like and used preferably. Particularly preferred is capsule, especially soft capsule. The capsule base material is not particularly limited, and gelatin derived from beef bones, cattle skin, pig skin, fish skin and the like, and other base materials (e.g., gum stabilizers that can be used as food additives, such as seaweed-derived products (e.g., carageenan, alginic acid and the like), vegetable seed-derived products (e.g., locust bean gum, guar gum and the like), and agents for production (e.g., celluloses) and the like) can also be used.

When processing the reduced coenzyme Q₁₀-containing composition of the present invention into the above-mentioned oral administration form or other form, other materials may be appropriately added depending on the object thereof. The above-mentioned other materials are not particularly limited and, for example, excipient, disintegrant, lubricant, binder, antioxidant, coloring agent, anticoagulant, absorption promoter, dissolution aids of the active ingredient, stabilizer and the like can be mentioned. It is needless to say that an active ingredient other than coenzyme Q₁₀ may also be co-present.

While the above-mentioned excipient is not particularly limited, for example, sucrose, lactose, glucose, cornstarch, mannitol, crystalline cellulose, calcium phosphate, calcium sulfate and the like can be used.

While the above-mentioned disintegrant is not particularly limited, for example, starch, agar, calcium citrate, calcium carbonate, sodium hydrogencarbonate, dextrin, crystalline cellulose, carboxymethylcellulose, tragacanth and the like can be used.

While the above-mentioned lubricant is not particularly limited, for example, talc, magnesium stearate, polyethylene glycol, silica, hydrogenated vegetable oil and the like can be used.

While the above-mentioned binder is not particularly limited, for example, ethylcellulose, methylcellulose, hydroxypropylmethylcellulose, tragacanth, shellac, gelatin, gum arabic, polyvinylpyrrolidone, polyvinyl alcohol, polyacrylic acid, polymethacrylic acid, sorbitol and the like can be used.

While the above-mentioned antioxidant is not particularly limited, for example, ascorbic acid, tocopherol, vitamin A, β-carotene, sodium hydrogensulfite, sodium thiosulfate, sodium pyrrosulfite, citric acid and the like can be used.

While the above-mentioned coloring agent is not particularly limited, for example, those allowed to be added to pharmaceutical products and food and the like can be used.

While the above-mentioned anticoagulant is not particularly limited, for example, stearic acid, talc, light anhydrous silicic acid, water-containing silicon dioxide and the like can be mentioned.

While the above-mentioned absorption promoter is not particularly limited, for example, higher alcohols, higher fatty acids, sucrose fatty acid ester, surfactants such as sorbitan fatty acid ester, sorbitan polyoxyethylene fatty acid ester and the like, and the like can be used.

While the dissolution aids for the above-mentioned active ingredient is not particularly limited, for example, organic acids such as fumaric acid, succinic acid, malic acid and the like, and the like can be mentioned.

While the above-mentioned stabilizer is not particularly limited, for example, benzoic acid, sodium benzoate, ethyl parahydroxybenzoate and the like can be mentioned.

The active ingredient other than the above-mentioned coenzyme Q₁₀ is, for example, amino acid, vitamin, mineral, polyphenol, organic acid, saccharides, peptide, protein and the like.

While the amount of the reduced coenzyme Q₁₀ contained in the composition of the present invention is not particularly limited, the weight of the reduced coenzyme Q₁₀ contained in the whole composition is generally not less than about 0.01 wt%, preferably not less than about 0.1 wt%, more preferably not less than about 1 wt%, particularly preferably not less than about 2 wt%, more preferably not less than about 3 wt%. While the upper limit is not particularly limited, it is generally not more than about 70%, preferably not more than about 60 wt%, more preferably not more than about 50 wt% in consideration of the viscosity of the composition and the like.

The production method of the crystal and composition of the present invention characteristically include a step of removing impurities by chromatography. As the impurities that can be removed by chromatography, cis-reduced coenzyme Q₁₀, reduced coenzyme Q₁₁, ubichromenol and the like can be mentioned. It is needless to say that the impurities other than the above-mentioned may also be removed by the chromatography.

In other embodiment, the production method of the present invention is a production method of reduced coenzyme Q₁₀, which comprises a step of purifying reduced coenzyme Q₁₀ containing at least one kind of cis-reduced coenzyme Q₁₀, reduced coenzyme Q₁₁, or ubichromenol as impurity by chromatography.

While the chromatography is not particularly limited, for example, column chromatography, thin layer chromatography and the like can be mentioned, with preference given to column chromatography.

While the carrier to be used for chromatography is not particularly limited, commercially available silica gel and the like can be used. The silica gel to be used as the carrier may be chemically modified. Examples of the silica gel include silica gel chemically modified with octadecyl group, silica gel chemically modified with octyl group, silica gel chemically modified with butyl group, silica gel chemically modified with trimethyl group, silica gel chemically modified with phenyl group and the like. Preferred is silica gel chemically modified with octadecyl group.

Reduced coenzyme Q₁₀ is easily oxidized upon contact with silica gel in the air. Thus, chromatography is desirably performed under an environment where reduced coenzyme Q₁₀ is protected from oxidation.

In one example, oxygen contained in a developing solvent is removed and chromatography is performed under a deoxygenation atmosphere. Removal of oxygen from a developing solvent and deoxygenation atmosphere can be achieved by substitution with inert gas, reduced pressure, boiling and combination of these. Use of at least the substitution with inert gas, i.e., inert gas atmosphere, is preferable. Examples of the above-mentioned inert gas include nitrogen gas, helium gas, argon gas, hydrogen gas, carbon dioxide gas and the like, with preference given to nitrogen gas.

It is also possible to prevent oxidation of reduced coenzyme Q₁₀ by the co-presence of a developing solvent and a strong acid having pKa of 2.5 or below, citric acids, ascorbic acids and the like. It is more preferable to combine the above-mentioned deoxygenation atmosphere and the above-mentioned co-presence of acids.

The developing solvent to be used for chromatography is not particularly limited, and only needs to be selected according to the reduced coenzyme Q₁₀ analog to be removed. Examples of such developing solvent include hydrocarbons, fatty acid esters, nitriles, ethers, ketones, alcohols, fatty acids and a mixed solvent thereof and the like.

While hydrocarbons are not particularly limited, for example, aliphatic hydrocarbon, aromatic hydrocarbon, halogenated hydrocarbon and the like can be recited. Particularly, aliphatic hydrocarbon and aromatic hydrocarbon are preferable, and aliphatic hydrocarbon is especially preferable.

Aliphatic hydrocarbon may be cyclic or non-cyclic, saturated or unsaturated and is not particularly limited. Generally, aliphatic hydrocarbon having 3 to 20 carbon atoms, preferably 5 to 12 carbon atoms, can be used.

Specific examples include propane, butane, isobutane, pentane, 2-methylbutane, cyclopentane, 2-pentene, hexane, 2-methylpentane, 2,2-dimethylbutane, 2,3-dimethylbutane, methylcyclopentane, cyclohexane, 1-hexene, cyclohexene, heptane, 2-methylhexane, 3-methylhexane, 2, 3-dimethylpentane, 2,4-dimethylpentane, methylcyclohexane, 1-heptene, octane, 2,2,3-trimethylpentane, isooctane, ethylcyclohexane, 1-octene, nonane, 2,2,5-trimethylhexane, 1-nonene, decane, 1-decene, p-menthane, undecane, dodecane and the like.

Of these, pentane, 2-methylbutane, cyclopentane, hexane, 2-methylpentane, 2,2-dimethylbutane, 2,3-dimethylbutane, methylcyclopentane, cyclohexane, heptane, 2-methylhexane, 3-methylhexane, 2,3-dimethylpentane, 2,4-dimethylpentane, methylcyclohexane, octane, 2,2,3-trimethylpentane, isooctane and ethylcyclohexane are preferably used.

While aromatic hydrocarbon is not particularly limited, normally, aromatic hydrocarbon having 6 to 20 carbon atoms, particularly 6 to 12 carbon atoms, especially 7 to 10 carbon atoms, is preferably used.
Specific examples include benzene, toluene, xylene, o-xylene, m-xylene, p-xylene, ethylbenzene, cumene, mesitylene, tetralin, butylbenzene, p-cymene, cyclohexylbenzene, diethylbenzene, pentylbenzene, dipentylbenzene, dodecylbenzene, styrene and the like. It is preferably toluene, xylene, o-xylene, m-xylene, p-xylene, ethylbenzene, cumene, mesitylene, tetralin, butylbenzene, p-cymene, cyclohexylbenzene, diethylbenzene or pentylbenzene, more preferably, toluene, xylene, o-xylene, m-xylene, p-xylene, cumene or tetralin, and most preferably cumene.

Halogenated hydrocarbon may be cyclic or non-cyclic, saturated or unsaturated, and is not particularly limited. In general, non-cyclic one is preferably used. Normally, chlorinated hydrocarbon and fluorinated hydrocarbon are preferable, and chlorinated hydrocarbon is particularly preferable. A halogenated hydrocarbon having 1 to 6 carbon atoms, particularly 1 to 4 carbon atoms, especially 1 or 2 carbon atoms, is preferably used.

Specific examples include dichloromethane, chloroform, carbon tetrachloride, 1,1-dichloroethane, 1,2-dichloroethane, 1,1,1-trichloroethane, 1,1,2-trichloroethane, 1,1,1,2-tetrachloroethane, 1,1,2,2-tetrachloroethane, pentachloroethane, hexachloroethane, 1,1-dichloroethylene, 1,2-dichloroethylene, trichloroethylene, tetrachloroethylene, 1,2-dichloropropane, 1,2,3-trichloropropane, chlorobenzene, 1,1,1,2-tetrafluoroethane and the like.

It is preferably dichloromethane, chloroform, carbon tetrachloride, 1,1-dichloroethane, 1,2-dichloroethane, 1,1,1-trichloroethane, 1,1,2-trichloroethane, 1,1-dichloroethylene, 1,2-dichloroethylene, trichloroethylene, chlorobenzene or 1,1,1,2-tetrafluoroethane, more preferably dichloromethane, chloroform, 1,2-dichloroethylene, trichloroethylene, chlorobenzene or 1,1,1,2-tetrafluoroethane.

While fatty acid esters are not particularly limited, for example, propionic acid ester, acetic acid ester, formic acid ester and the like can be used. Particularly, acetic acid ester and formic acid ester are preferable, and acetic acid ester is especially preferable. While ester group is not particularly limited, in general, alkyl ester or aralkyl ester having 1 to 8 carbon atoms, preferably alkyl ester having 1 to 6 carbon atoms, more preferably alkyl ester having 1 to 4 carbon atoms, is preferably used.

Examples of propionic acid ester include methyl propionate, ethyl propionate, butyl propionate and isopentyl propionate.

Examples of acetic acid ester include methyl acetate, ethyl acetate, propyl acetate, isopropyl acetate, butyl acetate, isobutyl acetate, sec-butyl acetate, pentyl acetate, isopentyl acetate, sec-hexyl acetate, cyclohexyl acetate, benzyl acetate and the like. It is preferably methyl acetate, ethyl acetate, propyl acetate, isopropyl acetate, butyl acetate, isobutyl acetate, sec-butyl acetate, pentyl acetate, isopentyl acetate, sec-hexyl acetate or cyclohexyl acetate, more preferably methyl acetate, ethyl acetate, propyl acetate, isopropyl acetate, butyl acetate or isobutyl acetate, and most preferably ethyl acetate.

Examples of formic acid ester include methyl formate, ethyl formate, propyl formate, isopropyl formate, butyl formate, isobutyl formate, sec-butyl formate, pentyl formate and the like. It is preferably methyl formate, ethyl formate, propyl formate, butyl formate, isobutyl formate or pentyl formate, and most preferably ethyl formate.

Nitriles may be cyclic or non-cyclic, saturated or unsaturated, and is not particularly limited. In general, saturated one is preferably used. Normally, nitrile having 2 to 20 carbon atoms, particularly 2 to 12 carbon atoms, especially 2 to 8 carbon atoms, is preferably used.

Specific examples include acetonitrile, propionitrile, malononitrile, butyronitrile, isobutyronitrile, succinonitrile, valeronitrile, glutaronitrile, hexanenitrile, heptyl cyanide, octyl cyanide, undecanenitrile, dodecanenitrile, tridecanenitrile, pentadecanenitrile, stearonitrile, chloroacetonitrile, bromoacetonitrile, chloropropionitrile, bromopropionitrile, methoxyacetonitrile, cyanomethyl acetate, cyanoethyl acetate, tolunitrile, benzonitrile, chlorobenzonitrile, bromobenzonitrile, cyanobenzoic acid, nitrobenzonitrile, anisonitrile, phthalonitrile, bromotolunitrile, methylcyanobenzoate, methoxybenzonitrile, acetylbenzonitrile, naphtonitrile, biphenylcarbonitrile, phenylpropionitrile, phenylbutyronitrile, methylphenylacetonitrile, diphenylacetonitrile, naphthylacetonitrile, nitrophenylacetonitrile, chlorobenzyl cyanide, cyclopropanecarbonitrile, cyclohexanecarbonitrile, cycloheptanecarbonitrile, phenylcyclohexanecarbonitrile, tolylcyclohexanecarbonitrile and the like.

It is preferably acetonitrile, propionitrile, succinonitrile, butyronitrile, isobutyronitrile, valeronitrile, cyanomethyl acetate, cyanoethyl acetate, benzonitrile, tolunitrile or chloropropionitrile, more preferably acetonitrile, propionitrile, butyronitrile or isobutyronitrile, and most preferably acetonitrile.

Ethers are not particularly limited and may be cyclic or non-cyclic, saturated or unsaturated, and is not particularly limited. In general, saturated one is preferably used. Normally, ether having 3 to 20 carbon atoms, particularly ether having 4 to 12 carbon atoms, especially ether having 4 to 8 carbon atoms, is preferably used.

Specific examples include diethyl ether, methyl tert-butyl ether, dipropyl ether, diisopropyl ether, dibutyl ether, dihexyl ether, ethylvinyl ether, butylvinyl ether, anisole, phenetol, butylphenyl ether, methoxytoluene, dioxane, furan, 2-methylfuran, tetrahydrofuran, tetrahydropyran, ethylene glycol dimethyl ether, ethylene glycol diethyl ether, ethylene glycol dibutyl ether, ethylene glycol monomethyl ether, ethylene glycol monoethyl ether, ethylene glycol dibutyl ether and the like.

It is preferably diethyl ether, methyl tert-butyl ether, dipropyl ether, diisopropyl ether, dibutyl ether, dihexyl ether, anisole, phenetol, butylphenyl ether, methoxytoluene, dioxane, 2-methylfuran, tetrahydrofuran, tetrahydropyran, ethylene glycol dimethyl ether, ethylene glycol diethyl ether, ethylene glycol dibutyl ether, ethylene glycol monomethyl ether or ethylene glycol monoethyl ether, more preferably diethyl ether, methyl tert-butyl ether, anisole, dioxane, tetrahydrofuran, ethylene glycol monomethyl ether or ethylene glycol monoethyl ether, more preferably diethyl ether, methyl tert-butyl ether, anisole and the like, and most preferably methyl tert-butyl ether.

Ketones are not particularly limited, and ketone having 3 to 6 carbon atoms is generally preferable. Specific examples include acetone, methylethylketone, methylbutylketone, methylisobutylketone and the like, and particularly, acetone and methylethylketone are preferable, and acetone is particularly preferable.

Alcohols may be cyclic or non-cyclic, saturated or unsaturated, and is not particularly limited. In general, saturated one is preferably used. Normally, monovalent alcohol having 1 to 20 carbon atoms, particularly 1 to 12 carbon atoms, especially 1 to 6 carbon atoms, particularly having 1 to 5 carbon atoms, particularly 1 to 3 carbon atoms, especially 2 or 3 carbon atoms, divalent alcohol having 2 to 5 carbon atoms or trivalent alcohol having 3 carbon atoms is preferably used.

Examples of monovalent alcohol include methanol, ethanol, 1-propanol, 2-propanol, 1-butanol, 2-butanol, isobutyl alcohol, tert-butyl alcohol, 1-pentanol, 2-pentanol, 3-pentanol, 2-methyl-1-butanol, isopentyl alcohol, tert-pentyl alcohol, 3-methyl-2-butanol, neopentyl alcohol, 1-hexanol, 2-methyl-1-pentanol, 4-methyl-2-pentanol, 2-ethyl-1-butanol, 1-heptanol, 2-heptanol, 3-heptanol, 1-octanol, 2-octanol, 2-ethyl-1-hexanol, 1-nonanol, 1-decanol, 1-undecanol, 1-dodecanol, allyl alcohol, propargyl alcohol, benzyl alcohol, cyclohexanol, 1-methylcyclohexanol, 2-methylcyclohexanol, 3-methylcyclohexanol, 4-methylcyclohexanol and the like.

It is preferably methanol, ethanol, 1-propanol, 2-propanol, 1-butanol, 2-butanol, isobutyl alcohol, tert-butyl alcohol, 1-pentanol, 2-pentanol, 3-pentanol, 2-methyl-1-butanol, isopentyl alcohol, tert-pentyl alcohol, 3-methyl-2-butanol, neopentyl alcohol, 1-hexanol, 2-methyl-1-pentanol, 4-methyl-2-pentanol, 2-ethyl-1-butanol or cyclohexanol, more preferably methanol, ethanol, 1-propanol, 2-propanol, 1-butanol, 2-butanol, isobutyl alcohol, tert-butyl alcohol, 1-pentanol, 2-pentanol, 3-pentanol, 2-methyl-1-butanol, isopentyl alcohol, tert-pentyl alcohol, 3-methyl-2-butanol or neopentyl alcohol, more preferably methanol, ethanol, 1-propanol, 2-propanol, 1-butanol, 2-butanol, isobutyl alcohol, 2-methyl-1-butanol or isopentyl alcohol, particularly preferably methanol, ethanol, 1-propanol or 2-propanol, further preferably ethanol, 1-propanol or 2-propanol, and most preferably ethanol.

Examples of divalent alcohol include 1,2-ethanediol, 1,2-propanediol, 1,3-propanediol, 1,2-butanediol, 1,3-butanediol, 1,4-butanediol, 2,3-butanediol, 1,5-pentanediol and the like. It is preferably 1,2-ethanediol, 1,2-propanediol or 1,3-propanediol, most preferably 1,2-ethanediol.

As trivalent alcohol, glycerol and the like can be preferably used.

Examples of fatty acids include formic acid, acetic acid, propionic acid and the like. It is preferably formic acid or acetic acid, most preferably acetic acid.

Of the above-mentioned solvents, use of a mixed solvent of at least two kinds selected from hydrocarbons, alcohols and fatty acids is preferable for removal of cis-reduced coenzyme Q₁₀, and use of a mixed solvent of each one kind from hydrocarbons, alcohols and fatty acids is particularly preferable. Use of a mixed solvent of hexane, 2-propanol and acetic acid is most preferable. When used as a mixed solvent, the solvent ratio of the mixed solvent is not particularly limited and can be appropriately set in consideration of the silica gel to be used, separability between reduced coenzyme Q₁₀ and impurities to be removed and the like. For example, when the above-mentioned mixed solvent of hexane, 2-propanol and acetic acid is used, the ratio of 2-propanol in the whole solvent is not more than 5%, more preferably not more than 3%, particularly preferably not more than 1%, in the volume ratio, and the ratio of acetic acid in the whole solvent is preferably not more than 5%, more preferably not more than 3%, particularly preferably not more than 1%, in the volume ratio.

For removal of ubichromenol and/or reduced coenzyme Q₁₁, use of a mixed solvent of at least two kinds selected from hydrocarbons, fatty acid esters and ethers is preferable, and use of a mixed solvent of hydrocarbons and alcohols is particularly preferable. Particularly, use of any one of hexane, methanol and ethanol is preferable, and most preferred is a mixed solvent of hexane and methanol or a mixed solvent of methanol and ethanol. When used as a mixed solvent, the solvent ratio of the mixed solvent is not particularly limited and can be appropriately set in consideration of the silica gel to be used, separability between reduced coenzyme Q₁₀ and impurities to be removed and the like. For example, when a mixed solvent of hexane and methanol is used, the ratio of methanol in the whole solvent is preferably not less than 80%, more preferably not less than 85%, particularly preferably not less than 90%, in the volume ratio. When a mixed solvent of methanol and ethanol is used, the ratio of methanol in the whole solvent is not particularly limited but preferably not less than 40%, more preferably not less than 45%, particularly preferably not less than 50%, in a volume ratio.

It is needless to say that an embodiment wherein two or more kinds of reduced coenzyme Q₁₀ analogs are removed by a single operation of chromatography is also encompassed in the present invention.

Another embodiment of the production method of the crystal or composition of the present invention comprises purifying oxidized coenzyme Q₁₀ containing at least one kind of cis-oxidized coenzyme Q₁₀, oxidized coenzyme Q₁₁, or ubichromenol as impurity by chromatography, and converting the purified oxidized coenzyme Q₁₀ to reduced coenzyme Q₁₀. To be specific, a production method.of a reduced coenzyme Q₁₀ crystal or reduced coenzyme Q₁₀-containing composition comprising subjecting oxidized coenzyme Q₁₀ containing at least one kind of cis-oxidized coenzyme Q₁₀, oxidized coenzyme Q₁₁ and ubichromenol (including a mixture of oxidized coenzyme Q₁₀ and reduced coenzyme Q₁₀) to chromatography to remove these impurities, which is followed by reduction to give high quality reduced coenzyme Q₁₀ is provided.

Since the operation of purifying oxidized coenzyme Q₁₀, or oxidized coenzyme Q₁₀ and reduced coenzyme Q₁₀ by chromatography, and converting the purified oxidized coenzyme Q₁₀ to reduced coenzyme Q₁₀ does not require strict oxidation protection conditions during the chromatography step, more convenient and economical production of high quality reduced coenzyme Q₁₀ can be afforded.

The carriers that can be used for the chromatography in this case are the same as those mentioned above.

As the developing solvent, the aforementioned developing solvents can be used. For removal of cis-oxidized coenzyme Q₁₀, use of a mixed solvent of at least two kinds selected from hydrocarbons, alcohols, fatty acid esters and fatty acids is preferable, and use of a combination of hydrocarbons and alcohols or hydrocarbons and fatty acid ester is particularly preferable. Specifically, a mixed solvent of two or more kinds selected from hexane, 2-propanol and ethyl acetate is preferable, and a mixed solvent of hexane and 2-propanol or a mixed solvent of hexane and ethyl acetate is more preferable. The solvent ratio of the mixed solvent is not particularly limited and can be appropriately set in consideration of the silica gel to be used, the separability between reduced coenzyme Q₁₀ and impurities to be removed and the like. For example, when the above-mentioned mixed solvent of hexane and 2-propanol is used, the ratio of 2-propanol in the whole solvent is preferably not more than 5%, more preferably not more than 3%, particularly preferably not more than 1%, in the volume ratio. In addition, when the above-mentioned mixed solvent of hexane and ethyl acetate is used, the ratio of ethyl acetate in the whole solvent is preferably not more than 5%, more preferably not more than 3%, and particularly preferably not more than 1%.

For removal of ubichromenol and/or reduced coenzyme Q₁₁, use of a mixed solvent of at least two kinds selected from hydrocarbons, fatty acid esters and ethers is preferable, and use of a mixed solvent of hydrocarbons and alcohols is more preferable. Specifically, use of any one of hexane, methanol and ethanol is preferable, and use of a mixed solvent of hexane and methanol, or methanol and ethanol is most preferable. The solvent ratio of the mixed solvent is not particularly limited and can be appropriately set in consideration of the silica gel to be used, the separability between reduced coenzyme Q₁₀ and impurities to be removed and the like. For example, when the above-mentioned mixed solvent of hexane and methanol is used, the ratio of methanol in the whole solvent is preferably not less than 80%, more preferably not less than 85%, particularly preferably not less than 90%. In addition, when the above-mentioned mixed solvent of methanol and ethanol is used, the ratio of methanol in the whole solvent is preferably not less than 40%, more preferably not less than 45%, and particularly preferably not less than 50%, in the volume ratio.

The oxidized coenzyme Q₁₀, or a mixture of oxidized coenzyme Q₁₀ and reduced coenzyme Q₁₀, obtained by chromatography can be converted to high quality reduced coenzyme Q₁₀, for example, by reduction according to the method described in W003/6408.

The thus-obtained reduced coenzyme Q₁₀ can be processed as it is into high quality reduced coenzyme Q₁₀ by concentration, drying and the like, or crystallized according to the method described in WO03/6408, WO03/6409 and the like to give a substantially pure reduced coenzyme Q₁₀ crystal with a low reduced coenzyme Q₁₀ analog content. From the aspects of easy handing and higher quality, reduced coenzyme Q₁₀ crystal is more preferable.

To exert the effect of the invention to the maximum extent, for example, the method of the present invention is preferably performed and the composition of the present invention is preferably prepared and/or preserved under a deoxygenation atmosphere such as inert gas atmosphere (e.g., above-mentioned nitrogen atmosphere etc.) and the like. The above-mentioned processing and preservation after processing are also preferably performed under the above-mentioned deoxygenation atmosphere such as inert gas atmosphere and the like.

According to the present invention, reduced coenzyme Q₁₀ analog, which is generally difficult to remove, can be conveniently removed and high quality reduced coenzyme Q₁₀ can be economically obtained with good workability.

### Examples

The present invention is explained in more detail in the following by referring to Examples, which are not to be construed as limitative. The purity of reduced coenzyme Q₁₀, the content of reduced coenzyme Q₁₀ analogs and the like in the Examples do not define the limit values of the purity in the present invention or the upper limit values thereof.

### (Example 1)

Reduced coenzyme Q₁₀ (5 g) containing 1.2% ubichromenol was dissolved in hexane (10 g). Reduced coenzyme Q₁₀ was adsorbed onto silica gel modified with octadecyl group in a column, and developed with n-hexane/methanol (3/17) solution to give a fraction containing reduced coenzyme 0₁₀. All the solvents used were free of oxygen by repetition of reduced pressure and released pressure with nitrogen, and all the operations were performed under a nitrogen atmosphere. The solution was concentrated and dried to give reduced coenzyme Q₁₀ as crystals. The reduced coenzyme 0₁₀ contained 0.3% of ubichromenol.

### (Example 2)

Reduced coenzyme Q₁₀ (5 g) containing 2.1% cis-reduced coenzyme 0₁₀ was dissolved in hexane (10 g). Reduced coenzyme Q₁₀ was adsorbed onto silica gel packed in a column, and developed with n-hexane/isopropanol/acetic acid (95/0.5/0.5) solution to give a fraction containing reduced coenzyme Q₁₀. All the solvents used were free of oxygen by repetition of reduced pressure and released pressure with nitrogen, and all the operations were performed under a nitrogen atmosphere. The solution was concentrated and dried to give reduced coenzyme Q₁₀ as crystals. The reduced coenzyme Q₁₀ contained 0.6% of cis-reduced coenzyme Q₁₀.

### (Example 3)

Reduced coenzyme Q₁₀ (5 g) containing 1.2% reduced coenzyme Q₁₁ was dissolved in hexane (10 g). Reduced coenzyme Q₁₀ was adsorbed onto silica gel modified with octadecyl group in a column, and developed with ethanol/methanol (4/3) solution to give a fraction containing reduced coenzyme Q₁₀. All the solvents used were free of oxygen by repetition of reduced pressure and released pressure with nitrogen, and all the operations were performed under a nitrogen atmosphere. The solution was concentrated and dried to give reduced coenzyme Q₁₀ as crystals. The reduced coenzyme Q₁₀ contained 0.3% of reduced coenzyme Q₁₁.

### (Example 4)

Reduced coenzyme Q₁₀ (5 g) containing 1.2% ubichromenol and 1.2% reduced coenzyme Q₁₁ was dissolved in hexane (10 g). Reduced coenzyme Q₁₀ was adsorbed onto silica gel modified with octadecyl group in a column, and developed with hexane/methanol (1/9) solution to give a fraction containing reduced coenzyme Q₁₀. All the solvents used were free of oxygen by repetition of reduced pressure and released pressure with nitrogen, and all the operations were performed under a nitrogen atmosphere. The solution was concentrated and dried to give reduced coenzyme Q₁₀ as crystals. The reduced coenzyme Q₁₀ contained 0.3% of ubichromenol and 0.3% of reduced coenzyme Q₁₁.

### (Example 5)

Reduced coenzyme Q₁₀ (5 g) containing 1.2% ubichromenol was dissolved in hexane (10 g). Reduced coenzyme Q₁₀ was adsorbed onto silica gel modified with octyl group in a column, and developed with n-hexane/methanol (1/9) solution to give a fraction containing reduced coenzyme Q₁₀. All the solvents used were free of oxygen by repetition of reduced pressure and released pressure with nitrogen, and all the operations were performed under a nitrogen atmosphere. The solution was concentrated and dried to give reduced coenzyme Q₁₀ as crystals. The reduced coenzyme Q₁₀ contained 0.8% of ubichromenol.

### (Example 6)

Reduced coenzyme Q₁₀ (5 g) containing 1.4% ubichromenol was dissolved in hexane (10 g). Reduced coenzyme Q₁₀ was adsorbed onto silica gel modified with octadecyl group in a column, and developed with n-hexane/methanol (1/19) solution to give a fraction containing reduced coenzyme Q₁₀. All the solvents used were free of oxygen by repetition of reduced pressure and released pressure with nitrogen, and all the operations were performed under a nitrogen atmosphere. The solution was concentrated and dried to give reduced coenzyme 0₁₀ as crystals. The reduced coenzyme Q₁₀ contained 0.2% of ubichromenol.

### (Example 7)

Oxidized coenzyme Q₁₀ (5 g) containing 1.2% ubichromenol was dissolved in hexane (10 g). Oxidized coenzyme Q₁₀ was adsorbed onto silica gel modified with an octadecyl group in a column, and developed with n-hexane/methanol (1/9) solution to give a fraction containing oxidized coenzyme Q₁₀. The fraction was concentrated to give a solution (50 g) containing 4.8 g of oxidized coenzyme 0₁₀. The oxidized coenzyme Q₁₀ contained 0.4% of ubichromenol. 10% Aqueous sodium hyposulfite solution (50 g) was added thereto and a reduction reaction was performed under a nitrogen atmosphere. After 2 hr, the aqueous phase was removed from the reaction mixture and the hexane phase was washed with deaerated saturated saline (50 g). All the operations from the reduction reaction to washing of the hexane phase with water were performed under a nitrogen atmosphere. The hexane phase was concentrated and dried to give reduced coenzyme Q₁₀ as crystals. The reduced coenzyme 0₁₀ contained 0.4% of ubichromenol.

### (Comparative Example 1)

### removal of reduced coenzyme Q₁₁ by crystal precipitation

Reduced coenzyme Q₁₀ (5 g) containing 1.2% reduced coenzyme Q₁₁ was dissolved in ethanol (83 g). While maintaining the same temperature, water (5 g) was added. The ethanol solution was cooled to 2°C at a cooling rate of 10°C/hr with stirring to give a white slurry. The obtained slurry was filtered under reduced pressure, the wet crystals were washed with cold ethanol, cold water and cold ethanol in this order (temperature of cold solvent used for washing, 2°C) and dried under reduced pressure (20 - 40°C, 1 - 30 mmHg) to give white dry crystals (4.85 g). All the operations mentioned above were performed under a nitrogen atmosphere. The obtained reduced coenzyme Q₁₀ contained 1.2% of reduced coenzyme Q₁₁.

### (Comparative Example 2)

### Removal of ubichromenol by crystal precipitation

Reduced coenzyme Q₁₀ (5 g) containing 1.4% ubichromenol was dissolved in ethanol (83 g) and dissolved at 50°C. While maintaining the same temperature, water (5 g) was added. The ethanol solution was cooled to 2°C at a cooling rate of 10°C/hr with stirring to give a white slurry. The obtained slurry was filtered under reduced pressure, the wet crystals were washed with cold ethanol, cold water and cold ethanol in this order (temperature of cold solvent used for washing, 2°C) and dried under reduced pressure (20 - 40°C, 1 - 30 mmHg) to give white dry crystals (4.85 g). All the operations mentioned above were performed under a nitrogen atmosphere. The obtained reduced coenzyme Q₁₀ contained 1.1% of ubichromenol.

While some of the embodiments of the present invention have been described in detail in the above, those of ordinary skill in the art can enter various modifications and changes to the particular embodiments shown without substantially departing from the novel teaching and advantages of the present invention. Such modifications and changes are encompassed in the spirit and scope of the present invention as set forth in the appended claims.

This application is based on application No. 2006-126900 filed in Japan, the contents of which are incorporated hereinto by reference.

## Claims

1. A substantially pure reduced coenzyme Q₁₀ crystal wherein the content of at least one kind of reduced coenzyme Q₁₀ analog selected from the group consisting of cis-reduced coenzyme Q₁₀, reduced coenzyme Q₁₁, and ubichromenol is not more than 1 wt%.

2. The reduced coenzyme Q₁₀ crystal of claim 1, wherein the content of cis-reduced coenzyme Q₁₀ is not more than 1 wt%.

3. The reduced coenzyme Q₁₀ crystal of claim 1, wherein the content of reduced coenzyme Q₁₁ is not more than 1 wt%.

4. The reduced coenzyme Q₁₀ crystal of claim 1, wherein the content of ubichromenol is not more than 1 wt%.

5. The reduced coenzyme Q₁₀ crystal of any one of claims 1 to 4, wherein the total content of cis-reduced coenzyme Q₁₀, reduced coenzyme Q₁₁, and ubichromenol is not more than 3 wt%.

6. The reduced coenzyme Q₁₀ crystal of claim 1, wherein the content of each of cis-reduced coenzyme Q₁₀, reduced coenzyme Q₁₁, and ubichromenol is not more than 1 wt%.

7. A reduced coenzyme Q₁₀-containing composition wherein the content of at least one kind of reduced coenzyme Q₁₀ analog selected from the group consisting of cis-reduced coenzyme Q₁₀, reduced coenzyme Q₁₁, and ubichromenol is not more than 1 wt%.

8. The reduced coenzyme Q₁₀-containing composition of claim 7, wherein the content of cis-reduced coenzyme Q₁₀ is not more than 1 wt%.

9. The reduced coenzyme Q₁₀-containing composition of claim 7, wherein the content of reduced coenzyme Q₁₁ is not more than 1 wt%.

10. The reduced coenzyme Q₁₀-containing composition of claim 7, wherein the content of ubichromenol is not more than 1 wt%.

11. The reduced coenzyme Q₁₀-containing composition of any one of claims 7 to 10, wherein the total content of cis-reduced coenzyme Q₁₀, reduced coenzyme Q₁₁, and ubichromenol is not more than 3 wt%.

12. The reduced coenzyme Q₁₀ crystal of claim 7, wherein the content of each of cis-reduced coenzyme Q₁₀, reduced coenzyme Q₁₁, and ubichromenol is not more than 1 wt%.

13. A production method of the reduced coenzyme Q₁₀ crystal of claim 1 or the reduced coenzyme Q₁₀-containing composition of claim 7, which comprises a step for removing impurities by chromatography.

14. The production method of claim 13, wherein the chromatography is column chromatography.

15. The production method of claim 13 or 14, wherein a carrier of the chromatography is silica gel or chemically-modified silica gel.

16. The production method of claim 15, wherein the chemically-modified silica gel is silica gel modified by an octadecyl group.

17. The production method of any one of claims 13 to 16, which comprises a step of purifying reduced coenzyme Q₁₀ containing at least one kind of cis-reduced coenzyme Q₁₀, reduced coenzyme Q₁₁, or ubichromenol as impurity by chromatography.

18. The production method of claim 17, wherein the chromatography is performed under an environment where reduced coenzyme Q₁₀ is protected from oxidation.

19. The production method of claim 17 or 18, wherein cis-reduced coenzyme Q₁₀ is removed using a mixed solvent of hexane, 2-propanol and acetic acid as a developing solvent.

20. The production method of claim 17 or 18, wherein the developing solvent is any one of hexane, methanol, ethanol and a mixed solvent thereof, and reduced coenzyme Q₁₁ and/or ubichromenol are/is removed.

21. The production method of any one of claims 13 to 16, which comprises purifying oxidized coenzyme Q₁₀ containing at least one kind of cis-oxidized coenzyme Q₁₀, oxidized coenzyme Q₁₁, or ubichromenol as impurity by chromatography, and converting the purified oxidized coenzyme Q₁₀ to reduced coenzyme Q₁₀.

22. The production method of claim 21, wherein the cis-oxidized coenzyme Q₁₀ is removed using, as the developing solvent, a mixed solvent of two or more kinds selected from hexane, 2-propanol and ethyl acetate.

23. The production method of claim 21, wherein the oxidized coenzyme Q₁₁ and/or ubichromenol are/is removed using, as the developing solvent, any one of hexane, methanol, ethanol and a mixed solvent thereof.
